# EUROPEAN PATENT SPECIFICATION

(11) **EP 0 974 353 B1**
(45) Date of publication and mention of the grant of the patent: **12.01.2005**
(21) Application number: 99110376.3
(22) Date of filing: 28.05.1999
(51) Int. Cl.: A61K 31/195, A61K 31/38, A61P 31/04

(54) **The use of homocysteine derivatives for the treatment of bacterial infections**
Verwendung von Homocysteinderivaten zur Behandlung von bakteriellen Infektionen
Utilisation de dérivés de l'homocysteine pour le traitement des infections bacterielles

(30) Priority: 05.06.1998 IT MI981267
(43) Date of publication of application: 26.01.2000
(73) Proprietor: EDMOND PHARMA S.R.L., I-20151 Milano (IT)
(72) Inventor: Nicola, Massimo, 20037 Paderno Dugnano (MI) (IT); Inglesi, Marco, 20037 Paderno Dugnano (MI) (IT)
(74) Representative: Minoja, Fabrizio, Dr.

(56) References cited:
- FR-A- 2 561 105
- CHEMICAL ABSTRACTS, vol. 111, no. 3, 17 July 1989 Columbus, Ohio, US; abstract no. 17471, SCURI, R. ET AL: "Effect of erdosteine and its metabolites on tracheobronchial mucus production and transport" XP000856242 & DRUGS EXP. CLIN. RES. (1988), 14(11), 693-8,
- KIM S.-C. ET AL: "Clinical efficacy of erdosteine in patients with acute or chronic bronchitis. A randomized, double blind, comparative study vs. ambroxol." TUBERCULOSIS AND RESPIRATORY DISEASES, (1997) 44/6 (1296-1307)., XP002123428
- CIACCIA, A. (1) ET AL: "Protection of erdosteine on smoke-induced peripheral neutrophil dysfunction both in healthy and in bronchitic smokers." FUNDAMENTAL & CLINICAL PHARMACOLOGY, (1992) VOL. 6, NO. 8-9, PP. 375-382., XP000856232
- RICEVUTI G ET AL: "Influence of erdosteine, a mucolytic agent, on amoxycillin penetration into sputum in patients with an infective exacerbation of chronic bronchitis." THORAX, (1988 AUG) 43 (8) 585-90., XP000856214
- MARCHIONI, C. F. ET AL: "Evaluation of efficacy and safety of erdosteine in patients affected by chronic bronchitis during an infective exacerbation phase and receiving amoxycillin as basic treatment (ECOBES, European Chronic Obstructive Bronchitis Erdosteine Study)" INT. J. CLIN. PHARMACOL. THER. (1995), 33(11), 612-18, XP002123429
- DECHANT, KERRY L. ET AL: "Erdosteine" DRUGS (1996), 52(6), 875-881, XP000856217
- BRAGA, PIER CARLO (1) ET AL: "Effects of erdosteine and its metabolites on bacterial adhesiveness." ARZNEIMITTEL-FORSCHUNG, (APRIL, 1999) VOL. 49, NO. 4, PP. 344-350., XP002123430

## Description

The present invention relates to the use of homocysteine derivatives as the sole active principle for the preparation of medicaments for the treatment of bacterial infections.

More precisely, the present invention relates to the use of N(thiodiglycoyl) homocysteine, or pharmaceutically acceptable salts thereof as the sole active principle for the preparation of medicaments for the treatment of bacterial infections.

N-(Thiodiglycoyl)homocysteine, of formula (A) 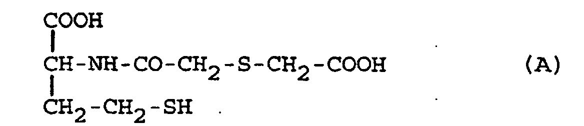 has been described by R. Scuri et al. in Drugs Exptl. Clin. Res., 1988, XIV(11), 693-698 as one of the active metabolites of the mucolytic agent N-(thiodiglycoyl)homocysteine gamma-thiolactone or erdosteine.

Furthermore, erdosteine, a metabolic precursor of compound (A), is known to be administered in combination with antibiotics and, when administered in combination with amoxicillin at doses of 1500 mg/day, it can give a synergistic effect (C.E. Marchioni et al., Intern. J. Clin. Pharmacol. Ther., 1995,33(11), 612-618).

It has now been found that N-(thiodiglycoyl)homocysteine (A) exerts a remarkable inhibitory activity on bacterial adhesiveness. Said inhibitory activity is apparently independent of the mucolytic activity, in that erdosteine and the other metabolites thereof are almost devoid of such an activity. Moreover, compound (A) is the erdosteine metabolite having the lowest mucolytic activity, as evidenced by R. Scuri et al. in the paper cited above.

Furthermore, it has been found that another known mucolytic agent, N-acetylcysteine, which has reportedly some activity on the inhibition of bacterial adhesiveness to epithelial cells of human mucosa (Niderman et al. Am. Rev. Resp. Di. 127, 85-90, 1983), is actually much less active, or even inactive, than compound (A), uner the same conditions.

Therefore, according to one of its aspects, the present invention relates to the use of N-(thiodiglycoyl)homocysteine, or pharmaceutically acceptable salts thereof for the preparation of medicaments for the treatment of bacterial infections.

Pharmaceutically acceptable salts are advantageously alkali or alkaline-earth salts, preferably the sodium, potassium or calcium salts.

Particularly advantageous homocysteine derivatives for use according to the invention are those compounds of formula (I) 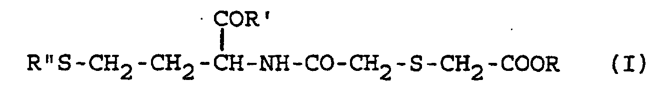 in which R is hydrogen or a (C₁-C₄)alkyl, R' is hydroxy or a (C₁-C₄)alkoxy group, R" is hydrogen or a (C₂-C₄)alkanoyl group or R' and R" are together a bond, as well as the pharmaceutically acceptable salts thereof at the free carboxylic groups.

Preferred compounds of formula (I) are those in which R is hydrogen or ethyl, R' is hydroxy or ethoxy and R" is hydrogen or acetyl, most preferred being the compound of formula (I) in which R is hydrogen and R' and R" together form a bond, and the pharmaceutically acceptable salts thereof at the free carboxylic groups.

The activity of compound of formula (A) was investigated in comparison with the other erdosteine metabolites described by R. Scuri et al. and with N-acetylcysteine, according to the procedure commonly used to verify the activity of antibiotics at concentrations lower than MIC (Minimal Inhibitory Concentration). Said procedures consist in incubating the active substance with bacteria in order to interfere with their adhesiveness mechanism and subsequently adding mucosa cells, observing the obtained result.

Human epithelial cells were collected from buccal and palatal mucosa of healthy volunteers by a sterile spatula, subsequently immersed in 2 ml of physiological saline buffered with phosphate buffer, combining then the suspensions from different volunteers.

Tests were carried out with three different *E. coli* strains, ATCC 25922 strain and two others isolated from human urinary infections, as well as with three *S. aureus* strains, ATCC 25923 strain and two others from human respiratory infections, using concentrations of 1.25, 2.5, 5 and 10 mcg/ ml for each tested compound.

At the end of the test, in the case of incubation with the three *E. coli* strains, the difference of activity between compound of formula (A) and the other tested compounds was found to be statistically significant, already at a concentration of 2.5 mcg/ml, i.e. whereas compound (A) showed a significant inhibitory activity of bacterial adhesiveness compared with the control (bacteria with no tested compound), N-acetylcysteine and one of erdosteine metabolites, N-acetylhomocysteine, showed no inhibitory activity on adhesiveness, even at the maximum tested concentration (10 mcg/ml) and the other erdosteine metabolite (homocysteine) showed some difference, which however was not statistically significant compared with the control test.

When incubating with S. *aureus,* N-acetylcysteine and the other erdosteine metabolites showed no specific activity in reducing bacterial adhesiveness, whereas compound (A), at concentrations of 2.5 to 10 mcg/ml, induced a significant reduction of S. *aureus* adhesiveness to epithelial cells.

In the case of erdosteine, as well, bacterial adhesiveness was not inhibited in a statistically significant way, but erdosteine (compound of formula (I), R = hydrogen, R' + R'' bond) is known to become active, also as a mucolytic agent, only after thiol groups have been released by tissutal enzymatic action (Medical Praxis, 1992, 13(3/4) 71-76) and to be the direct precursor metabolic of compound (A).

Thanks to the activity of compound (A) on bacterial adhesiveness, to the poor toxicity and to the excellent tolerability of the compound itself and of its metabolic precursors, these compounds, in particular compounds of formula (I) and the pharmaceutically acceptable salts thereof at the carboxylic groups, can be used, according to the invention, for the preparation of medicaments for inhibiting bacterial adhesiveness.

In particular, the medicaments containing compound (A) can replace antibiotics, at least in some pathologies of the respiratory tract or of genitourinary apparatus, in that they are intended for the first choice treatment of bacterial infections, specially of the respiratory tract. They thus allow, in many cases, to avoid antibiotic treatment. The present invention allows in any case to decrease the antibiotic doses or the duration of the treatment with the antibiotic itself.

The medicaments prepared using compound (A), the pharmaceutically acceptable salts thereof, can be formulated in pharmaceutical compositions containing 4 to 2000 mg, advantageously 50 to 1000 mg, preferably 100 to 500 mg, of active ingredient per dosage unit, for the oral or rectal administration or for inhalation 1 to 3 times a day. In the pharmaceutical compositions, the active ingredient is in admixture with conventional pharmaceutical excipients.

Some compounds of formula (I) are novel and are particularly interesting precursors of compound (A).

Therefore, according to a further aspect, the invention relates to novel thiol derivatives of formula (I) 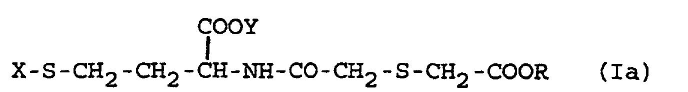 in which R and Y are independently hydrogen or a (C₁-C₄)alkyl, X is hydrogen or a (C₂-C₄)alkanoyl group, at least one thereof being different from hydrogen, and the pharmaceutically acceptable salts thereof at the free carboxylic groups.

Among these novel compounds, particularly advantageous are those of formula (Ia) in which one of R and Y is hydrogen and the other is a (C₁-C₄)alkyl, preferably ethyl, and X is hydrogen or a (C₂-C₄)alkanoyl, preferably acetyl, as well as those of formula (Ia) in which both R and Y are hydrogen and X is (C₂-C₄)alkanoyl and the pharmaceutically acceptable salts thereof.

The novel compounds of formula (Ia) in which Y is hydrogen can be obtained starting from an erdosteine (C₁-C₄)alkyl ester which is treated with an alkali base, such as sodium hydroxide, to obtain the compound of formula (Ia), in which X is hydrogen, Y is an alkali metal, such as sodium, and R is (C₁-C₄)alkyl. This compound is neutralized to give the compound of formula (Ia) in which X and Y are hydrogen and R is (C₁-C₄)alkyl. The resulting compound can be treated with a (C₂-C₄)alkanoic acid functional derivative to obtain a compound of formula (Ia) in which Y is hydrogen, R is (C₁-C₄)alkyl and X is (C₂-C₄)alkanoyl.

The compounds of formula (Ia) in which R is hydrogen can be obtained starting from a homocysteine (C₁-C₄)alkyl ester which is treated with 3,5-dioxo-[1,4lthioxane in alkali medium. The compound of formula (Ia) in which R and X are hydrogen and Y is (C₁-C₄)alkyl is obtained by acidification. The resulting compound can be treated with a (C₂-C₄)alkanoic acid functional derivative to obtain a compound of formula (Ia) in which R is hydrogen, Y is (C₁-C₄)alkyl and X is (C₂-C₄)alkanoyl.

Compounds of formula (Ia) in which both R and Y are a (C₁-C₄)alkyl group can be prepared by esterification of the corresponding monoesters, thus giving asymmetric esters.

Compounds of formula (Ia) in which both R and Y are hydrogen and X is (C₂-C₄)alkanoyl can be prepared by treatment of compound (A) with a (C₂-C₄)alkanoic acid functional derivative, preferably acetic acid.

The following examples further illustrate the invention.

### PREPARATION I - Compound (A), disodium salt

24.93 g of erdosteine (Il Farmaco Ed. Sci., 1986, XLI(1), 69-79) are added with 95.6 ml of a 2.09 M solution of sodium hydroxide, under stirring, to complete dissolution, keeping stirring for 4 hours under nitrogen atmosphere. The solution is evaporated to obtain N-(thiodiglycoyl)homocysteine disodium (formula (I), R= Na, R'= ONa, R'' = H) as a hygroscopic solid.
IR (nujol) ν = 3287, 1785, 1741, 1675 and 1611 cm⁻¹.

### PREPARATION II - Compound (A)

An aqueous solution of 10 g of the disodium salt obtained in PREPARATION I is extracted with ethyl acetate to remove any impurities, then the aqueous phase is acidified to acid pH with 1N hydrochloric acid, then saturated with sodium chloride and extracted again with ethyl acetate. The organic phase is dried over anhydrous sodium sulfate and after filtering off the resulting solid, it is evaporated to dryness. 9.5 g of N-(thiodiglycolyl)homocysteine are obtained (formula (I), R = R'' = H, R' = OH); m.p. 144 - 146°C.
IR (nujol) ν = 3287, 1785, 1741, 1675 and 1611 cm⁻¹.

### EXAMPLE 1

A suspension of 4 g of homocysteine ethyl ester in 50 ml of water is added with 5N sodium hydroxide to pH 7.5, then a solution of 2.65 g of 3,5-dioxo-[1,4]-thioxane in 30 ml of tetrahydrofuran is added dropwise in 30 minutes, keeping pH at 7.5 by addition of 5N sodium hydroxide. The mixture is acidified to pH 1 with 5N hydrochloric acid, then extracted with ethyl acetate and the organic phase is dried over sodium sulfate and evaporated to dryness. The solid is crystallized from ethanol/ether isopropyl (1/1) to give 1.29 g of ethyl N-[(2-carboxymethylthio)acetyl]homocysteinate (formula (Ia), R = X = H, Y = ethyl); m.p. 137- 139°C.
IR (nujol) ν = 3290, 1740, 1685, 1629 and 1561 cm⁻¹.

### EXAMPLE 2

A solution of 1.39 g of ethyl N-[(2-carboxymethylthio)acetyl]homocysteinate in 20 ml of dichloromethane is added with 1.3 ml of tetrahydrofuran and the resulting mixture is added with 0.33 ml of acetyl chloride with cooling. The mixture is stirred at room temperature for 60 minutes, then washed with water and the organic phase is dried over anhydrous sodium sulfate, then concentrated to dryness to obtain an oily residue which slowly cystallizes to give 0.5 g of ethyl S-acetyl-N-[(2-carboxymethylthio)acetyl]omocysteinate (formula (Ia), R = H, X = acetyl, Y = ethyl): m.p. 72 - 75°C.
IR (nujol) ν = 3229, 2929, 1781, 1676 and 1536 cm⁻¹.

### EXAMPLE 3

A suspension of 15 g of erdosteine ethyl ester (Il Farmaco Ed. Sci., 1986, XLI(I), 69-79) in 24.12 ml of a 2.24N sodium hydroxide solution is kept 7 days under stirring at room temperature. The solid is filtered off, the residual aqueous solution is treated with 1N hydrochloric acid to pH 1, saturated with sodium chloride and extracted with ethyl acetate. The organic phase is dried over anhydrous sodium sulfate, then evaporated to dryness. 8.5 g of N-[2-(ethoxycarbonylmethylthio)acetyl]homocysteine are obtained (formula (Ia), R = ethyl, Y = X = H) as oily liquid.
IR (neat) ν = 3319, 2642, 2642, 1727, 1632 and 1537 cm⁻¹.

### EXAMPLE 4

A solution of 3.1 g of N-[2-(ethoxycarbonylmethylthio)acetyl]homocysteine in 25 ml of dimethoxyethane is added dropwise with 2.91 ml of triethylamine and 0.74 ml of acetyl chloride in 30 minutes, cooling at 0 - 5°C, then the mixture is stirred for 60 minutes at room temperature and concentrated to dryness. The residue is taken up into water, the mixture is acidified to pH 1 with 1N hydrochloric acid and finally extracted with ethyl acetate. The organic phase is dried over anhydrous sodium sulfate and concentrated to dryness to give 2.65 g of S-acetyl-N-[2-(ethoxycarbonylmethylthio)acetyl]homocysteine (formula (Ia), R = ethyl, Y = H, X = acetyl), as an oily residue which solidifies; m.p. 140 - 143°C.
IR (KBr) ν = 3290, 1712, 1689, 1630 and 1561 cm⁻¹.

## Claims

1. The use of a homocysteine derivative of formula 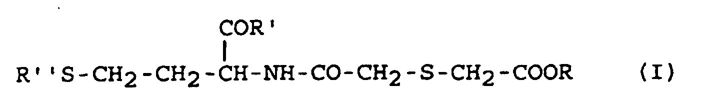 in which R is hydrogen or a (C₁-C₄)alkyl, R' is hydroxyl or a (C₁-C₄)alkoxy group, R" is hydrogen or a (C₂-C₄)alkanoyl group or R' and R" together form a bond, or a pharmaceutically acceptable salt thereof at the free carboxylic groups as the sole active principle in the preparation of medicaments for the treatment of bacterial infections.

2. The use as claimed in claim 1, wherein bacterial infections are respiratory tract infections.

3. The use as claimed in claim 1, wherein bacterial infections are genitourinary tract infections.

4. The use as claimed in claims 1-3, wherein said pharmaceutically acceptable salts are the alkali or alkaline-earth salts.

5. The use as claimed in claims 1-4, wherein said homocysteine derivative is a compound of formula (I) in which R is hydrogen or ethyl, R' is hydroxyl or ethoxy, R" is hydrogen or acetyl or a pharmaceutically acceptable salt thereof at the free carboxylic groups.

6. The use as claimed in claim 5, wherein said homocysteine derivative is a compound of formula (I) in which R is hydrogen and R' and R" together form a bond, or a pharmaceutically acceptable salt thereof.

## Patentansprüche

1. Verwendung eines Homocysteinderivats der Formel (I) 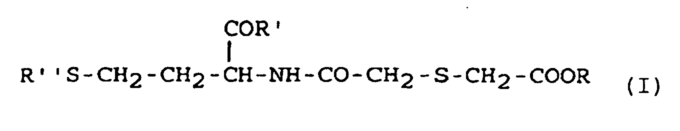 worin R Wasserstoff oder eine (C₁-C₄)Alkylgruppe ist, R' Hydroxyl oder eine (C₁-C₄)Alkoxygruppe ist, R" Wasserstoff oder eine (C₂-C₄)Alkanoylgruppe ist oder R' und R" zusammen eine Bindung bilden, oder eines pharmazeutisch annehmbaren Salzes davon an den freien Carbonsäuregruppen als das einzige aktive Prinzip in der Herstellung von Medikamenten zur Behandlung bakterieller Infektionen.

2. Verwendung nach Anspruch 1, wobei bakterielle Infektionen Respirationstraktinfektionen sind.

3. Verwendung nach Anspruch 1, wobei bakterielle Infektionen Urogenitaltraktinfektionen sind.

4. Verwendung nach den Ansprüchen 1 bis 3, wobei die pharmazeutisch annehmbaren Salze die Alkali- oder Erdalkalisalze sind.

5. Verwendung nach den Ansprüchen 1 bis 4, wobei das Homocysteinderivat eine Verbindung der Formel (I), worin R Wasserstoff oder Ethyl ist, R' Hydroxyl oder Ethoxy ist, R'' Wasserstoff oder Acetyl ist, oder ein pharmazeutisch annehmbares Salz davon an den freien Carbonsäuregruppen ist.

6. Verwendung nach Anspruch 5, wobei das Homocysteinderivat eine Verbindung der Formel (I), in der R Wasserstoff ist und R' und R" zusammen eine Bindung bilden, oder ein pharmazeutisch annehmbares Salz davon ist.

## Revendications

1. Utilisation d'un dérivé d'homocystéine de formule 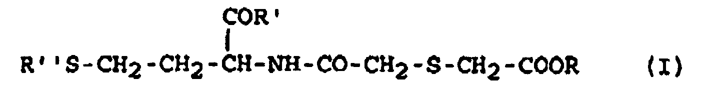 dans laquelle R est de l'hydrogène ou un groupe alkyle en C₁ à C₄, R' est un groupe hydroxyle ou alcoxy en C₁ à C₄, R" est de l'hydrogène ou un groupe alcanoyle en C₂ à C₄ ou R' et R" forment ensemble une liaison,
ou d'un sel pharmaceutiquement acceptable de celui-ci aux groupes carboxyliques libres, en tant qu'unique principe actif dans la préparation de médicaments pour le traitement d'infections bactériennes.

2. Utilisation selon la revendication 1, dans laquelle les infections bactériennes sont des infections des voies respiratoires.

3. Utilisation selon la revendication 1, dans laquelle les infections bactériennes sont des infections de l'appareil génito-urinaire.

4. Utilisation selon les revendications 1 à 3, dans laquelle lesdits sels pharmaceutiquement acceptables sont les sels alcalins ou alcalino-terreux.

5. Utilisation selon les revendications 1 à 4, dans laquelle ledit dérivé d'homocystéine est un composé de formule (1) dans laquelle R est de l'hydrogène ou un groupe éthyle, R' est un groupe hydroxyle ou éthoxy, R" est de l'hydrogène ou un groupe acétyle, ou un sel pharmaceutiquement acceptable de celui-ci aux groupes carboxyliques libres.

6. Utilisation selon la revendication 5, dans laquelle ledit dérivé d'homocystéine est un composé de formule (I) dans laquelle R est de l'hydrogène, et R' et R" forment ensemble une liaison, ou un sel pharmaceutiquement acceptable de celui-ci.
